# FASCICULE DE BREVET EUROPEEN

(11) **EP 1 540 289 B1**
(45) Date de publication et mention de la délivrance du brevet: **20.07.2016**
(21) Numéro de dépôt: 03769581.4
(22) Date de dépôt: 05.09.2003
(51) Int. Cl.: G01F 1/704, G21H 5/02

(54) **PROCEDE ET DISPOSITIF POUR LA DETERMINATION EN CONTINU DE LA CONSOMMATION EN HUILE LUBRIFIANTE D'UN MOTEUR A COMBUSTION INTERNE**
VERFAHREN UND VORRICHTUNG ZUR KONTINUIERLICHEN BESTIMMUNG DES ÖLVERBRAUCHS EINER BRENNKRAFTMASCHINE
METHOD AND DEVICE FOR THE CONTINUOUS DETERMINATION OF THE LUBRICATING OIL CONSUMPTION OF AN INTERNAL COMBUSTION ENGINE

(30) Priorité: 06.09.2002 FR 0211050
(43) Date de publication de la demande: 15.06.2005
(73) Titulaire: TOTAL MARKETING SERVICES, 92800 Puteaux (FR); Delta Services Industriels SPRL, 7503 Froyennes (BE)
(72) Inventeur: DELVIGNE, Thierry, B-7503 Froyennes (BE); OBIOLS, Jérôme, F-69003 Lyon (FR)
(74) Mandataire: Radault, Gabrielle
(86) Numéro de dépôt international: PCT/FR2003/002657
(87) Numéro de publication internationale: WO 2004/023084

(56) Documents cités:
- FR-A- 2 307 257
- GB-A- 2 051 354
- US-A- 2 939 011
- US-A- 2 957 986
- US-A- 3 471 696
- BERGMANN M ET AL: "METHODE ZUR OELVERBRAUCHMESSUNG DURCH MARKIERUNG MIT RADIOAKTIVEM BROM", MTZ MOTORTECHNISCHE ZEITSCHRIFT, VIEWEG VERLAG, WIESBADEN, DE, vol. 58, no. 2, 1 February 1997 (1997-02-01), pages 102-107, XP000678384, ISSN: 0024-8525

## Description

La présente invention concerne un procédé et un dispositif pour la détermination en continu de la consommation d'un moteur à combustion interne en huile lubrifiante. Ce procédé utilise un radiotraceur incorporé dans l'huile et dont la quantité mesurée dans les gaz d'échappement est proportionnelle à la consommation d'huile moteur.

On sait l'importance que présente aussi bien pour les fabricants d'automobiles que pour les producteurs d'huiles lubrifiantes et/ou d'additifs la connaissance précise de la consommation des moteurs de véhicule en huile lubrifiante, que ce soit pour prévenir une usure prématurée des pièces du moteur ou pour faire fonctionner celui-ci dans les meilleures conditions de lubrification.

A ce jour, diverses méthodes ont été proposées pour mesurer la consommation des moteurs à combustion interne en huile lubrifiante, mais elles présentent toutes l'inconvénient d'être discontinues.

Elles comportent, en outre, divers désavantages suivant leur nature, par exemple celui de nécessiter un appareil de mesure très important, dans le cas de l'utilisation d'un traceur au brome radioactif, de conduire à des résultats non fiables du fait d'une altération par le traceur lui-même, dans le cas de l'utilisation de traceurs au soufre ou au zinc, voire même d'imposer d'effectuer les mesures dans un laboratoire muni d'équipements spéciaux de sécurité, si l'on emploie du tritium comme traceur.

Du fait de ces inconvénients, on a donc suggéré de marquer les huiles avec un traceur radioactif et de mesurer la quantité de ce traceur présente dans les gaz d'échappement, en faisant barboter ceux-ci dans une solution aqueuse d'acide nitrique et de nitrate d'argent (voir "A Method of Measuring Oil Consumption by Labelling with Radioactive Bromine", H. Zellbeck, M. Bergmann, J. Rôthig, J. Seibold et A. Zeuner, Tribotest Journal 6-3, Mars 2000 ou encore « Methode zur Olverbrauchmessung durch Markierung mit radioaktivem Brom», M. Bergmann et al, MTZ Motortechnische Zeitschrift 58 (1997) 2).

On a également proposé d'incorporer à l'huile un traceur radioactif à base de brome, par exemple le 1,2-dibromooctadécane, et de mesurer la teneur des gaz d'échappement en ce composé à l'aide d'une solution basique, par exemple de soude (Brevet U.S. N° 3 471 696).

L'inconvénient de ces méthodes est qu'elles impliquent des opérations de montage et de démontage du dispositif d'analyse et que les mesures effectuées sont échelonnées sur une durée assez longue.

Le document US 2 957 986 propose également d'incorporer à l'huile un traceur radioactif, notamment le carbone 14, et de mesurer la teneur des gaz d'échappement en traceur en installant un détecteur sur la conduite de gaz d'échappement. Lorsque la quantité de traceur n'est pas mesurable par le détecteur, les gaz d'échappement traversent un absorbeur (par exemple de l'hydroxyde de baryium) afin de faire précipiter le CO₂. Le précipité ainsi obtenu sur une période de temps donnée est ensuite chauffé pour libérer le CO₂ et effectuer la mesure de carbone 14 sur le gaz libéré. Cette méthode implique donc également des opérations de montage et de démontage du dispositif d'analyse.

On ressent donc le besoin, dans la technique, de disposer d'un procédé et d'un dispositif permettant la mesure en continu de la consommation par un moteur à combustion interne d'huile lubrifiante, sans avoir à monter ou démonter des dispositifs spéciaux, qui puissent être mis en oeuvre facilement à l'aide d'appareillages simples et éprouvés, et qui n'affectent en rien les propriétés de l'huile concernée.

Dans son principe, l'invention consiste à utiliser dans ce but la présence dans l'huile lubrifiante d'un traceur radioactif incorporé à celle-ci, pour mesurer en aval du moteur la radioactivité des gaz de combustion à l'aide d'une sonde sensible à un rayonnement ionisant et en déduire la consommation du moteur en huile lubrifiante.

L'invention a par conséquent pour premier objet un procédé de détermination en continu de la consommation d'un moteur à combustion interne en huile lubrifiante selon la revendication 1.

Le traceur radioactif, choisi tel que son rayonnement soit mesurable, peut être de nature très diverse et est sélectionné parmi des espèces activables Ei et/ou des espèces Eii radioactives de par leur nature.

On notera que la quantité nécessaire de traceur radioactif contenue dans l'huile lubrifiante dépendra notamment de la nature du traceur (activité, type et énergie de rayonnement), du positionnement du détecteur par rapport au piège, de la géométrie du détecteur et du piège et des éventuels blindages.

Selon les cas, les espèces Ei sont activées soit avant leur incorporation au sein de l'huile moteur ou soit au sein de l'huile moteur. Cette activation est effectuée par voie neutronique via une irradiation effectuée par une source neutronique ou effectuée par un faisceau de protons au moyen d'un accélérateur de particules, dans des conditions appropriées connues de l'homme du métier.

Une des options possibles pour l'activation est d'incorporer l'espèce Ei à une quantité appropriée d'un vecteur (par exemple, des solvants et/ou diluants tels qu'une huile de dilution), puis de soumettre le mélange obtenu à l'activation adéquate et enfin de l'ajouter à l'huile moteur.

Ces espèces Ei susceptibles d'être marquées englobent notamment les éléments suivants : le zinc, le brome, le sodium, le molybdène, le phosphore, le soufre, le cuivre, le calcium et le magnésium, et des composés comprenant ces éléments.

A titre d'exemples de composés utilisables d'espèces Ei, on citera les familles d'additifs usuels pour lubrifiants suivants : dithiophosphate de zinc, sulfonates de calcium, sulfonates de magnésium, phénates de calcium, phénates de magnésium, salicylates de calcium, salicyclates de magnésium, etc.

On peut également utiliser d'autres espèces Ei sans impact sur les propriétés d'utilisation de l'huile et dont la quantité recueillie au niveau de la sortie des gaz d'échappement reste corrélée à la consommation d'huile moteur.

Pour les espèces Eii, on citera, par exemple, les isotopes des halogènes tels que, par exemples, le brome-82, le technétium 99-m, le strontium-85, le germanium-68, le germanium-69 et le cobalt-56.

Comme pour les espèces Ei, les éléments naturellement radioactifs peuvent être utilisés seuls ou sous forme de composés contenant ledit élément, et être incorporés ou non au sein d'un vecteur (par exemple, des solvants et/ou diluants, tels qu'une huile de dilution). Par exemple, le technétium 99-m peut être incorporé dans l'huile sous forme d'une solution aqueuse de pertechnétate de sodium NaTcO₄.

On peut également utiliser du technétium 99-m conditionné sous forme de particules de dimensions nanométriques et isolées de l'atmosphère par du carbone ; on citera à ce sujet le produit commercialisé sous la marque Technégaz (ce produit est habituellement utilisé pour l'étude clinique de l'aération des poumons.)

Concernant l'utilisation du germanium-68 ou du germanium-69, on peut également ajouter à l'huile au moins un tétra-alkyl germane contenant au moins l'un de ces deux isotopes. La longueur des chaînes alkyles de ces tétra-alkyl germanes étant proportionnelle à leur point d'ébullition, on utilisera avantageusement un mélange de tétra-alkyl germanes dont les points d'ébullition sont représentatifs de l'intervalle de distillation du lubrifiant considéré. A titre d'exemples, le tétrahexylgermane, tétraheptylgermane et tétraoctylgermane ont des points d'ébullition comparable à un lubrifiant moteur classique.

Afin de simplifier l'élimination des traceurs radioactifs retenus par le piège, on utilise de préférence des éléments radioactifs à courte demi-vie tels que le brome 82, le technétium 99-m, le germanium-69 etc... Le technétium 99-m est particulièrement préféré à cause de sa très courte demi-vie (6 heures) et de la disparition très rapide de sa radioactivité, au bout d'environ 3 jours.

Le piège apte à retenir physiquement le radiotraceur de l'huile lubrifiante peut être de différents types. En général, le piège comporte au moins un élément de filtration constitué par au moins un support filtrant à structure poreuse fixé dans une enveloppe métallique qui est reliée à la ligne des gaz d'échappement. Le support filtrant ou les éléments filtrants disposés dans l'enveloppe métallique du filtre (appelée "canning") peuvent être constitués par des éléments en céramique poreuse. Le support filtrant est traversé par les gaz d'échappement entre une extrémité d'entrée et une extrémité de sortie du filtre, ce qui permet de retenir les particules de traceur(s) radioactif(s) du lubrifiant contenues dans les gaz d'échappement.

Dans le cadre de l'invention, on utilisera avantageusement les filtres à particules utilisés par les constructeurs d'automobiles pour éliminer les composés organiques et le carbone contenus dans les gaz d'echappement.

On notera que le détecteur de(s) traceur(s) radioactif(s) retenu(s) par le piège peut être disposé avantageusement à proximité immédiate de celui-ci, ce qui facilite considérablement les mesures.

Ce détecteur est une sonde de détection de rayonnements ionisants (rayons bêta, X ou gamma) pouvant être soit de type scintillateur liquide ou solide [cristal iodure de sodium NaI(Tl), cristal BGO] ou soit de type semi-conducteur [cristal germanium, cristal CZT].

Ces types de détecteurs permettent de mettre en oeuvre de façon continue le procédé conforme à l'invention et l'acquisition des données peut se faire en un temps très court, de l'ordre d'une seconde.

On notera, en outre, que le détecteur peut déceler simultanément la présence de divers traceurs marqués de l'huile ainsi que leurs quantités respectives dans les gaz de combustion.

Les signaux détectés sont ensuite traités par une série de moyens permettant de calculer la consommation du moteur en huile lubrifiante ; ces moyens comprennent notamment un moyen de traitement du signal détecté (par exemple, amplificateur, filtre et convertiseur analogique/digital CAD), un moyen d'analyse des hauteurs d'impulsions (par exemple, analyseur multicanal) et un moyen de stockage et de traitement des données acquises (par exemple, ordinateur PC).

L'invention a également pour objet un dispositif pour la détermination en continu de la consommation d'un moteur à combustion interne en huile lubrifiante selon la revendication 7.

Le piège apte à retenir les particules de(s) traceur(s) radioactif(s) de l'huile marquée peut être placé en aval du moteur en toute position lui permettant d'être au contact des gaz de combustion. Il peut ainsi être situé sur la ligne d'échappement des gaz du moteur ou sur une dérivation prévue à cet effet.

Avant rejet à l'atmosphère des gaz d'échappement, si le piège à particules n'est pas lui-même un filtre à particules, un tel filtre devra nécessairement être prévu en aval de ce piège, sur la ligne d'échappement.

Les dessins annexés illustrent la mise en oeuvre de l'invention. Sur ces dessins :
La figure 1 est une vue schématique illustrant le procédé de l'invention ;
Les figures 2 et 3 sont des diagrammes relatifs à des exemples de mise en oeuvre qui seront décrits ci-après.

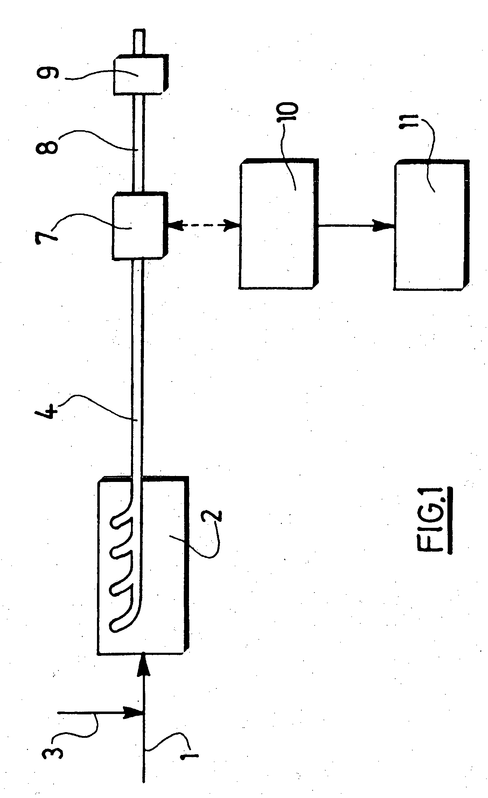

On se référera d'abord à la figure 1.

En dérivation sur le circuit d'huile lubrifiante 1 du moteur à combustion interne 2 est prévue une ligne 3 d'introduction d'une quantité prédéterminée de la même huile lubrifiante additionnée d'au moins un traceur radioactif et permettre de mesurer la consommation en huile du moteur.

Les produits de combustion du moteur 2 sont évacués par la ligne 4 jusqu'à un piège 7, apte à retenir physiquement les particules du traceur radiochimique ou de l'additif radioactivé présentes dans les gaz d'échappement.

Avant d'être évacués à l'extérieur par la ligne 8, ces gaz traversent un filtre 9, destiné à retenir les dernières particules radioactives présentes.

A proximité immédiate du piège 7 est prévue une sonde 10 de détection de rayonnements ionisants, qui permet de mesurer en continu la quantité présente dans le piège 7 du radiotraceur incorporé dans l'huile lubrifiante ou du ou des éléments marqués par activation d'additifs contenus dans cette huile.

On notera que l'activation par neutrons thermiques et/ou faisceau de protons des espèces Ei n'affecte en rien les caractéristiques de l'huile lubrifiante, car les neutrons thermiques sont des particules très peu énergétiques.

Ainsi qu'il a été exposé ci-dessus, on utilisera de préférence, comme radiotraceur de l'huile lubrifiante, un composé radioactif à courte demie-vie, notamment le technétium 99m.

Les exemples ci-après, qui n'ont pas de caractère limitatif, illustrent la mise en oeuvre de l'invention et les avantages de celle-ci.

### Exemples

Ces exemples sont destinés à illustrer la mesure de la consommation par un moteur thermique à quatre temps d'une huile lubrifiante marquée à l'aide d'un traceur radioactif, qui est piégé par un filtre à particules placé sur le circuit d'échappement du moteur.

Le moteur quatre temps utilisé pour les exemples 1 et 2 est un moteur de motocyclette HONDA, connu sous l'appellation commerciale HORNET, de 600 cm³.

Le moteur utilisé dans l'exemple 3 est un moteur 2.2L turbo-diesel, équipant un véhicule Renault Laguna.

L'huile pour moteur à quatre temps utilisée dans les exemples 1 et 2 est une huile pour motocyclette commercialisée sous la marque ELF, type 4 DXRatio. L'huile utilisée dans l'exemple 3 est une huile pour moteur automobile, commercialisée sous la marque ELF Prestigrade 15W40.

On a utilisé les deux traceurs suivants ;
- l'isotope ^{99M}Tc, disponible sous forme de pertechnétate de sodium, NaTc O₄, en solution aqueuse,
- et l'isotope radioactif ⁶⁵Zn, obtenu par irradiation d'un additif connu et usuel pour une huile lubrifiante, un dithiophosphate de zinc secondaire (noté DTPZn), qui contient une quantité élevée de zinc (plus de 10% en poids), que l'on soumet à une irradiation pendant plusieurs heures sous un haut flux de neutrons par un réacteur nucléaire, qui transforme le ⁶⁴Zn stable en ⁶⁵Zn radioactif.

Le piège à particules radioactives utilisé est un filtre à particules disponible dans le commerce, installé sur les véhicules Peugeot équipés du moteur 2,2 litres HDI.

Le système de détection des particules radioactives retenues par le piège est un détecteur standard NaI(Tl) de 3*3 pouces avec tube photomultiplicateur intégré, les autres éléments de la chaîne de mesure étant un préamplificateur de charge modèle 2007P de marque Canberra, un amplificateur de spectroscopie 2020 (Canberra), un convertisseur ADC modèle 8087 (Canberra), et une carte multicanaux modèle S100 (Canberra). Les logiciels mis en oeuvre lors de ces essais sont « Génie 2000 » (Canberra) pour la spectrométrie gamma, ainsi que le logiciel d'analyse MCS (Multi Channel Scaling) « IDSWear » commercialisé par la société Atlantic Nuclear Services (ANS), Canada.

### Exemple 1

Cet exemple concerne la mesure de la consommation de l'huile en utilisant comme traceur le ^{99M}Tc.

La solution aqueuse de pertechnétate de sodium est miscible en petite quantité (2 à 3% en poids, selon le type d'huile) avec l'huile pour moteur quatre temps.

On part d'une solution aqueuse de NaTcO₄ ayant une activité spécifique de 500 MBq/ml (Méga Becquerels par millilitre).

On prélève 2,22 ml de cette solution et on les mélange à 3 litres d'huile pour moteur quatre temps, pour aboutir à une activité spécifique de 370 MBq/1.

On introduit l'huile ainsi marquée dans le carter du moteur (moteur non contaminé, alimenté par une essence non marquée), on fait démarrer le moteur et on le fait fonctionner à différents régimes.

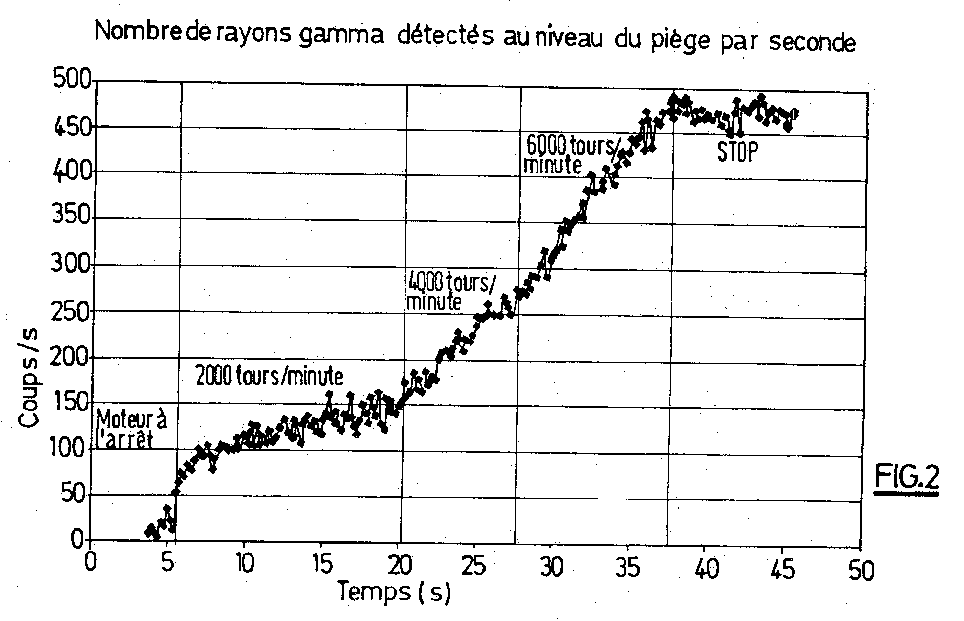

La figure 2 est un diagramme illustrant le nombre de rayons gamma détectés par seconde au niveau du filtre à particules, à différents régimes du moteur, en fonction du temps.

L'augmentation d'activité détectée en fonction du temps et des différents régimes du moteur (2000, 4000 et 6000 tours/mn) correspond à la consommation d'huile.

Cette consommation est faible sur ce type de moteur et très difficile à apprécier avec des méthodes de mesure usuelles, alors que le procédé conforme à l'invention se prête parfaitement à de telles mesures. De plus, le procédé permet le suivi en continu de la consommation d'huile.

On voit, sur la figure 2, que la pente de la courbe, qui reflète la consommation d'huile, croît avec le régime suivant une relation sensiblement linéaire dans cette plage de régimes. Cette pente est en effet, la suivante, en fonction du régime moteur :

| Tours par minute | Pente (coups/s) | Consommation |
|---|---|---|
| 2000 | 1,10 | 10,7 ml/h |
| 4000 | 2,67 | 25,9 ml/h |
| 6000 | 4,10 | 39,9 ml/h |

La prise en compte de la géométrie de détection et de l'efficacité de la chaîne de comptage permet d'estimer les consommations indiquées dans la colonne de droite pour chaque régime du moteur.

### Exemple 2.

Cet exemple concerne la consommation d'huile marquée au ⁶⁵Zn..

A partir d'un échantillon du DTPZn activé par irradiation neutronique, dont l'activité spécifique est de 95 kBq/ml, on prélève 30 ml que l'on mélange à 3 litres d'huile pour moteur quatre temps, pour aboutir à une activité spécifique de 950 kBq/1.

L'huile ainsi marquée est introduite dans le carter du moteur. Etant donné la faible activité spécifique du traceur disponible, la configuration du moteur a été modifiée pour cet essai, de manière à atteindre une consommation en huile élevée, voisine de 1 litre par heure à 6000 tours/mn.

Le moteur, non contaminé et alimenté avec de l'essence non activée, est mis en marche et on le fait fonctionner au régime stabilisé de 6000 tours/min.

Compte tenu de la longue demi-vie du ⁶⁵ Zn (244 jours), une seule série de mesures a été effectuée dans cet essai, afin de ne pas contaminer à l'excès l'ensemble de l'appareillage de mesure (banc d'essai et filtre à particules), le but étant simplement de tester le procédé conforme à l'invention sur un moteur dont la consommation en huile est nettement plus importante que dans l'Exemple 1, avec en outre un isotope autre que le ⁹⁹Tc.

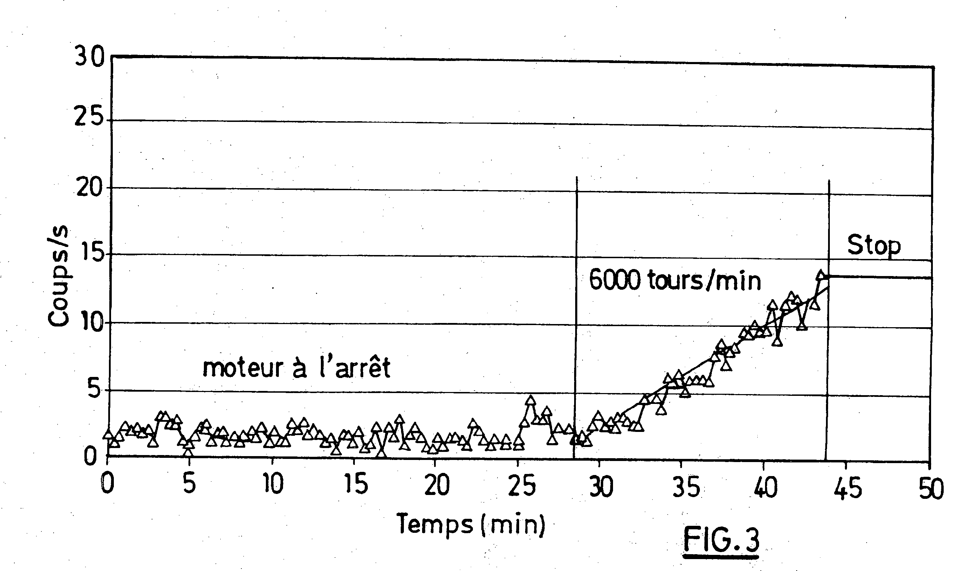

La figure 3 est un diagramme illustrant le nombre de rayons gamma détectés par seconde au niveau du filtre à particules, à 6000 tours par minutes, en fonction du temps.

On constate que la pente de la courbe est d'environ 0,73 coup/s. La prise en compte de la géométrie de détection et de l'efficacité de la chaîne de comptage pour le rayonnement émis par le ⁶⁵Zn (ce rayonnement est d'énergie nettement supérieure au ⁹⁹mTc) permet d'estimer la consommation du moteur à 0,87 litres par heure.

Les Exemples 1 et 2, mettent donc en évidence une relation entre les vitesses de rotation du moteur et la montée en activité du filtre à particules, qui sont cohérentes avec les consommations d'huile auxquelles on peut s'attendre dans ces conditions.

L'utilisation d'un isotope ne perturbe pas les mesures et l'on observe des comportements similaires avec deux isotopes différents.

### Exemple 3.

Cet exemple concerne la consommation d'huile marquée au ⁶⁵Zn.

A partir d'un échantillon du DTPZn activé par irradiation neutronique, dont l'activité spécifique est de 95 kBq/ml, on prépare 50 litres d'huile ayant une activité spécifique de 21.2 kBq/1.

L'huile ainsi marquée est introduite dans le carter du moteur. Le véhicule, installé sur un banc à rouleaux, suit des cycles pré-programmés de 471 km, choisis de manière à favoriser la consommation d'huile par le moteur.

Le moteur utilisé est non contaminé et est alimenté avec du carburant non activé. Six vidanges ont été effectuées après 10.000 km, et une vidange a été effectuée après 20.000 km. A chaque vidange, la consommation d'huile du moteur est calculée en faisant la différence entre la masse d'huile initiale et la masse d'huile de vidange, recueillie par pesée, à laquelle s'ajoute la masse des éventuels appoints d'huile effectués entre chaque vidange. L'augmentation de l'activité en Zn-65 du filtre à particules entre chaque vidange est également mesurée.

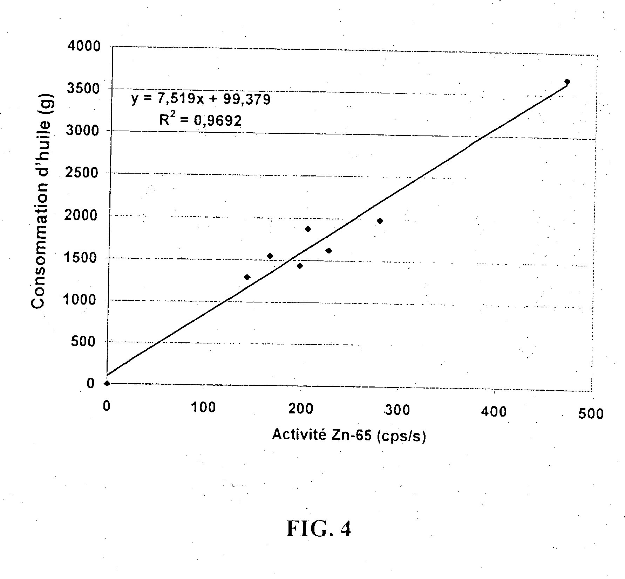

La figure 4 montre la corrélation entre la consommation d'huile du moteur à chaque vidange, calculée par pesée, et l'augmentation d'activité au niveau du filtre à particules.

Cet exemple montre donc que le suivi de l'accumulation de Zn-65, utilisé sous forme de DTPZn activé, au niveau du filtre à particules est représentatif de la consommation d'huile du moteur.

La mesure d'activité au niveau du filtre à particules étant possible en continu, il est donc possible de mesurer en continu la consommation d'huile du moteur en utilisant ce dispositif et ce procédé.

## Revendications

1. Procédé de détermination de la consommation en huile lubrifiante d'un moteur (2) à combustion interne, dans lequel :
- on marque l'huile lubrifiante dont on désire mesurer la consommation avec une quantité déterminée d'au moins un traceur radioactif ;
- on mesure en aval du moteur (2), dans les gaz issus de celui-ci, la quantité de(s) traceur(s) radioactif(s) présente ;
- et l'on en déduit la consommation du moteur en huile lubrifiante ;
la mesure dans les gaz issus du moteur (2) de la quantité présente de(s) traceur(s) radioactif(s) de l'huile lubrifiante comprenant :
- la mise en contact de ces gaz avec un piège (7) apte à retenir physiquement les particules de(s) traceur(s) radioactif(s), ce piège comportant au moins un élément de filtration formé par au moins un support filtrant à structure poreuse, fixé dans une enveloppe métallique;
- la mesure en continu du rayonnement en provenance du piège, à l'aide d'un détecteur (10) sensible au rayonnement émis par le(s) traceur(s) radioactif(s) retenu(s) par le piège (7) et placé à une distance de celui-ci permettant de mesurer le rayonnement émis ;
- et la transmission des mesures effectuées par ce détecteur (10) à un ordinateur programmé apte à convertir ces mesures en le taux de consommation du moteur en huile lubrifiante.

2. Procédé selon la revendication 1, **caractérisé en ce que** le traceur radioactif incorporé dans l'huile lubrifiante est un élément radioactif à courte demi-vie, notamment le brome 82 ou le technétium 99m.

3. Procédé selon la revendication 2, **caractérisé en ce que** le traceur radioactif est du technétium 99-m et est incorporé dans l'huile sous forme d'une solution aqueuse de pertechnétate de sodium NaTcO₄.

4. Procédé selon la revendication 2, **caractérisé en ce que** le traceur radioactif est du technétium 99-m et est incorporé dans l'huile sous forme de particules de dimensions nanométriques et isolées de l'atmosphère par du carbone.

5. Procédé selon la revendication 1, **caractérisé en ce que** le traceur radioactif incorporé dans l'huile lubrifiante est choisi parmi le germanium-68 et/ou le germanium-69, de préférence sous la forme d'au moins un tétra-alkyl germane contenant au moins le germanium-68 et/ou le germanium-69.

6. Procédé selon la revendication 1, caractérisé en ce le traceur radioactif est un élément ou un composé comprenant cet élément, qui a été activé par voie neutronique et/ou par un faisceau de protons avant incorporation dans cette huile.

7. Dispositif pour la détermination en continu de la consommation en huile lubrifiante d'un moteur (2) à combustion interne, ce dispositif comprenant :
- un moyen d'incorporation dans l'huile lubrifiante d'une quantité déterminée d'au moins un traceur radioactif ;
- des moyens pour mesurer en aval du moteur, dans les gaz de combustion issus de celui-ci, la quantité du traceur radioactif qui y est présente ;
- et des moyens pour déduire de cette mesure la consommation en huile du moteur ;
ce dispositif comprenant :
(i) en aval du moteur (2), un piège (7) avec lequel viennent en contact les gaz de combustion issus du moteur et qui est apte à retenir physiquement les particules de(s) traceur(s) radioactif(s) présentes dans ces gaz, ce piège (7) comportant au moins un élément de filtration formé par au moins un support filtrant à structure poreuse, fixé dans une enveloppe métallique ;
(ii) à proximité de ce piège (7) et à une distance de celui-ci permettant de mesurer un rayonnement émis par les particules de traceur(s) radioactif(s) retenues par ce piège, un détecteur (10) sensible à ce rayonnement ;
(iii) en liaison fonctionnelle avec le détecteur (10), un ordinateur programmé (11) apte à calculer à partir des informations relevées par le détecteur la consommation du moteur en huile lubrifiante ou en additif.

8. Dispositif selon la revendication 7, **caractérisé en ce que** le piège (7) est placé sur la ligne d'échappement des gaz de combustion du moteur (2) ou encore sur une dérivation prévue à cet effet.

9. Dispositif selon l'une quelconque des revendications 7 ou 8, **caractérisé en ce que** le piège (7) comprend un filtre à particules.

10. Dispositif selon l'une quelconque des revendications 7 à 9, **caractérisé en ce que** le détecteur (10) est une sonde de détection de rayonnements ionisants.

11. Dispositif selon l'une quelconque des revendications 7 à 10, **caractérisé en ce qu'**il comprend un filtre (9) disposé sur la ligne d'échappement des gaz de combustion entre le piège (7) et le point de rejet de ces gaz à l'atmosphère.

## Patentansprüche

1. Verfahren zur Bestimmung des Schmierölverbrauchs eines Verbrennungsmotors (2), worin:
- das Schmieröl, dessen Verbrauch gemessen werden soll, mit einer bestimmten Menge von mindestens einem radioaktiven Indikator markiert wird;
- dem Motor (2) nachgelagert, in den von diesem ausgestoßenen Gasen, die Menge des/der vorhandenen radioaktiven Indikators/Indikatoren gemessen wird;
- und daraus der Schmierölverbrauch des Motors hergeleitet wird;
wobei das Messen der in den von dem Motor (2) ausgestoßenen Gasen vorhandenen Menge an radioaktivem/radioaktiven Indikator(en) des Schmieröls umfasst:
- das Inkontaktbringen dieser Gase mit einer Falle (7), welche dazu geeignet ist, die Teilchen des/der radioaktiven Indikators/Indikatoren physisch zurückzuhalten, wobei die Falle mindestens ein Filtrationselement, gebildet aus mindestens einem Filterträger mit poröser Struktur, fixiert in einem metallischen Mantel, umfasst;
- das kontinuierliche Messen der von der Falle ausgehenden Strahlung mittels eines Detektors (10), empfindlich gegen die Strahlung, emittiert von dem/den radioaktiven Indikator(en), welche(r) von der Falle (7) zurückgehalten wird/werden, und platziert in einem Abstand von diesem, welcher das Messen der emittierten Strahlung gestattet;
- und das Übertragen der mit dem Detektor (10) durchgeführten Messungen an einen Rechner, geeignet programmiert, um diese Messwerte in die Verbrauchsrate des Motors an Schmieröl umzurechnen.

2. Verfahren nach Anspruch 1, **dadurch gekennzeichnet, dass** der radioaktive Indikator, welcher in das Schmieröl eingebracht ist, ein radioaktives Element mit kurzer Halbwertszeit ist, insbesondere Brom-82 oder Technetium-99m.

3. Verfahren nach Anspruch 2, **dadurch gekennzeichnet, dass** der radioaktive Indikator Technetium-99m ist und in das Öl in Form einer wässrigen Lösung von Natriumpertechneat NaTcO₄ eingebracht ist.

4. Verfahren nach Anspruch 2, **dadurch gekennzeichnet, dass** der radioaktive Indikator Technetium-99m ist und in das Öl in Form von Teilchen mit Dimensionen im Nanometerbereich, welche durch Kohlenstoff von der Atmosphäre isoliert sind, eingebracht ist.

5. Verfahren nach Anspruch 1, **dadurch gekennzeichnet, dass** der radioaktive Indikator, der in das Schmieröl eingebracht ist, aus Germanium-68 und/oder Germanium-69, vorzugsweise in Form mindestens eines Tetraalkylgermans, enthaltend mindestens das Germanium-68 und/oder das Germanium-69, ausgewählt ist.

6. Verfahren nach Anspruch 1, **dadurch gekennzeichnet, dass** der radioaktive Indikator ein Element oder eine das Element umfassende Verbindung ist, welches durch Neutronen und/oder durch einen Protonenstrahl vor dem Einbringen in das Öl aktiviert wurde.

7. Vorrichtung zur kontinuierlichen Bestimmung des Schmierölverbrauchs eines Verbrennungsmotors (2), wobei die Vorrichtung umfasst:
- ein Mittel zum Einbringen einer bestimmten Menge von mindestens einem radioaktiven Indikator in das Schmieröl;
- Mittel, um dem Motor nachgelagert, in den von diesem ausgestoßenen Verbrennungsgasen, die Menge des darin vorhandenen radioaktiven Indikators zu messen;
- und Mittel zum Herleiten des Ölverbrauchs des Motors aus dieser Messung;
wobei die Vorrichtung umfasst:
(i) dem Motor (2) nachgelagert, eine Falle (7) mit der die von dem Motor ausgestoßenen Verbrennungsgase in Kontakt treten und welche dazu geeignet ist, die in diesen Gasen vorhandenen Teilchen des/der radioaktiven Indikators/Indikatoren physisch zurückzuhalten, wobei die Falle (7) mindestens ein Filtrationselement, gebildet aus mindestens einem Filterträger mit poröser Struktur, fixiert in einem metallischen Mantel, umfasst;
(ii) in der Nähe dieser Falle (7) und mit einem Abstand zu ihr, welcher das Messen einer Strahlung, welche von den von der Falle zurückgehalten Teilchen des/der radioaktiven Indikators/Indikatoren emittiert wird, gestattet, einen Detektor (10), welcher gegen diese Strahlung empfindlich ist;
(iii) in funktioneller Verbindung mit dem Detektor (10) einen Rechner (11), geeignet programmiert, um, ausgehend von den Informationen, ermittelt durch den Detektor, den Schmieröl- oder Additivverbrauch des Motors zu berechnen.

8. Vorrichtung nach Anspruch 7, **dadurch gekennzeichnet, dass** die Falle (7) im Abgasweg der Verbrennungsgase des Motors (2) beziehungsweise in einer dafür vorgesehenen Abzweigung platziert ist.

9. Vorrichtung nach einem der Ansprüche 7 oder 8, **dadurch gekennzeichnet, dass** die Falle (7) einen Teilchenfilter umfasst.

10. Vorrichtung nach einem der Ansprüche 7 bis 9, **dadurch gekennzeichnet, dass** der Detektor (10) eine Sonde zur Detektion von ionisierender Strahlung ist.

11. Vorrichtung nach einem der Ansprüche 7 bis 10, **dadurch gekennzeichnet, dass** sie einen Filter (9) umfasst, welcher in dem Abgasweg der Verbrennungsgase zwischen der Falle (7) und dem Punkt des Ausstoßes dieser Gase an die Atmosphäre angebracht ist.

## Claims

1. A method for determination of the lubricating oil consumption of an internal combustion engine (2), in which:
- the lubricating oil whose consumption is to be measured is labeled with a determined quantity of at least one radioactive tracer;
- downstream of the engine (2), the quantity of radioactive tracer(s) present in the gases emerging from the latter is measured;
- and the lubricating oil consumption of the engine is deduced therefrom;
the measurement of the quantity of radioactive lubricating oil tracer(s) present in the gases emerging from the engine comprising:
- bringing these gases in contact with a trap (7) which can physically retain the radioactive tracer particles, this trap including at least one filtration element formed by at least one filtering medium with a porous structure, fixed in a metal canning;
- with the aid of a detector (10) sensitive to radiation emitted by the radioactive tracer(s) retained by the trap (7) and placed at a distance therefrom allowing the emitted radiation to be measured, continuously measuring this radiation coming from the trap;
- and transmitting the measurements taken by this detector (10) to a programmed computer which can convert these measurements into the lubricating oil consumption rate of the engine.

2. The method as claimed in claim 1, **characterized in that** the radioactive tracer incorporated into the lubricating oil is a radioactive element with a short half-life, in particular bromine 82 or technetium 99m.

3. The method as claimed in claim 2, **characterized in that** the radioactive tracer is technetium 99m and is incorporated into the oil in the form of an aqueous solution of sodium pertechnate NaTcO₄.

4. The method as claimed in claim 2, **characterized in that** the radioactive tracer is technetium 99m and is incorporated into the oil in the form of particles which have nanometric dimensions and are isolated from the atmosphere by carbon.

5. The method as claimed in claim 1, **characterized in that** the radioactive tracer incorporated into the lubricating oil is selected from germanium-68 and/or germanium-69, preferably in the form of at least one tetraalkyl germane containing at least germanium-68 or germanium-69.

6. The method as claimed in claim 1, **characterized in that** the radioactive tracer is an element, or a compound comprising this element, which has been neutron activated and/or activated by a proton beam before incorporation into this oil.

7. A device for the continuous determination of the lubricating oil consumption of an internal combustion engine (2), this device comprising:
- a means for incorporating a determined quantity of at least one radioactive tracer into the lubricating oil;
- means for measuring downstream of the engine, in the combustion gases emerging from the latter, the quantity of the radioactive tracer which is present therein;
- and means for deducing the lubricating oil consumption of the engine from this measurement;
this device comprising:
(i) downstream of the engine (2), a trap (7) with which the combustion gases emerging from the engine come in contact and which can physically retain the radioactive tracer particles, this trap including at least one filtration element formed by at least one filtering medium with a porous structure, fixed in a metal canning;
(ii) in proximity to this trap (7) and at a distance therefrom allowing radiation emitted by the radioactive tracer particles retained by this trap to be measured continuously and while the engine is in use and the trap is in contact with the gases emerging from the engine, a detector (10) sensitive to this radiation;
(iii) functionally linked to the detector (10), a programmed computer (11) which can calculate the lubricating oil or additive consumption of the engine on the basis of the information recorded by the detector.

8. The device as claimed in claim 7, **characterized in that** the trap (7) is placed on the combustion gas exhaust line of the engine (2), or on a branch line intended for this purpose.

9. The device as claimed in any one of claims 7 or 8, **characterized in that** the trap (7) comprises a particle filter.

10. The device as claimed in any one of claims 7 to 9, **characterized in that** the detector (10) is a probe for detection of ionizing radiation.

11. The device as claimed in any one of claims 7 to 10, which comprises a filter (9) arranged on the combustion gas exhaust line, between the trap (7) and the point where these gases are discharged to the atmosphere.
